Europäisches Patentamt

European Patent Office

Office européen des brevets

(19)

(11) Publication number:  0 153 536

A2

# (12) EUROPEAN PATENT APPLICATION

(21) Application number: 84308943.4

(22) Date of filing: 20.12.84

(51) Int. Cl.⁴: C 07 C 1/20
//B01J21/06

(30) Priority: 07.02.84 GB 8403156

(43) Date of publication of application:
04.09.85 Bulletin 85/36

(84) Designated Contracting States:
BE DE FR NL SE

(71) Applicant: Coal Industry (Patents) Limited
Hobart House Grosvenor Place
London SW1X 7AE(GB)

(72) Inventor: Robinson, Joseph Gordon
"Claybrook" The Hyde
Winchcombe Gloucestershire(GB)

(72) Inventor: Riemer, Pierce William Foster
27 Lambourne Avenue
Huntly Gloucestershire(GB)

(74) Representative: Wood, John Irwin
Hobart House Grosvenor Place
London SW1X 7AE(GB)

(54) Improvements in catalysts.

(57) It has been found that synthetic microporous catalyst which are active in converting oxygen-containing aliphatics into hydrocarbons can lose activity; deactivation of the catalyst can be reduced significantly by mixing an organic additive which contains ethylenic unsaturation or a precursor therefor with the feedstock.

EP 0 153 536 A2

Croydon Printing Company Ltd.

- 1 -

Case 4662

"IMPROVEMENTS IN CATALYSTS"

This invention concerns improvements in catalysts, more especially it concerns improvements in catalyst life for certain synthetic aluminosilicate catalysts.

We have, in British published Patent Application No. 2,100,710A, described a novel amorphous synthetic microporous catalyst which exhibits certain properties similar to Mobil's ZSM-5 catalyst, particularly the conversion of methanol or dimethyl ether into hydrocarbons boiling in the gasoline range although the product distribution varies from ZSM-5. Our co-pending British Patent Application, No. 83/30873, further describes a preferred protonic form of such catalysts, and other indications of utility in synthesis reactions.

Extended tests with such catalysts showed that overall conversion of a methanol feedstock under standard conditions declined from about 90% to about 50% after 15 hours on stream. Analysis of the products, which were principally mono-cyclic aromatic hydrocarbons, led to the observation that benzene was rarely found, and it was thought that benzene could be very reactive under the reaction conditions and in the presence of the highly porous and active catalyst. Possibly, therefore, it seemed that if benzene were present in excess, it could form substituted benzenes through the utilisation of the precursors which were thought to be responsible for coke formation and consequent catalyst deactivation. Tests were accordingly carried out using a mixture of methanol and benzene, but only a relatively modest increase in catalyst life was observed.

Alternative aromatic feedstock supplements also offered increased catalyst life, but we have now discovered a method offering significant improvements.

International Patent Application WO82/01866 describes the promotion of the conversion of methanol, especially methanol and water feedstocks, to olefins, lower alkanes and monocyclic aromatic hydrocarbons, over crystalline catalysts of the synthetic zeolite type, such as ZSM-5, using a promoter which is an aromatic hydrocarbon, a precursor to an aromatic hydrocarbon, an olefin or a precursor thereof or an aldehyde. It appears that the promoter improves the activity of the catalyst, and that aromatic hydrocarbons including especially benzene, toluene and p-xylene are the most active promoters. Included in the promoters tested were cyclohexene and ethylene, but the results were not as good as with those preferred compounds. This contrasts significantly with the findings on which the present invention are based and which are illustrated hereinafter, and points to a fundamentally different interaction between feedstock and catalyst.

The present invention provides a method for converting an oxygen-containing aliphatic feedstock into hydrocarbons, in the vapour phase, over a synthetic amorphous microporous catalyst, comprising the admixture of from 1 to 15% by weight of an organic additive which contains at least one olefinic linkage which is not deactivated, or a precursor thereof, with the oxygen-containing aliphatic feedstock, optionally with the addition of up to 20% by weight of water, based on the feedstock, whereby deactivation of the catalyst is reduced.

The additives preferably contain at least one olefinic linkage and have hydrogen or an alkyl, especially methyl, or aryl group on carbon. atoms adjacent to the linkage. Aromatic unsaturation or compounds with substituents which tend to accept electrons, are not, therefore, to be considered within the scope of the invention.

The additive is suitably an alkene or cycloalkene and may be selected for best results with the particular feedstock and catalyst and for the product distribution desired. Precursors for the olefinic organic compound are those substances which under the reaction conditions, and in the presence of the zeolite-type catalyst, yield such olefinic organic compounds; particularly to be considered are alcohols and ketones. Good results have been attained with cyclohexene, cyclohexadiene and 1-hexene; butadiene, cyclohexanone and cyclohexanol may be recommended. Also to be considered as additives are ethylene and ethylenic compounds which may be considered as having one to four methyl substituents in place of the hydrogen atoms.

The amount of additive is preferably from 3 to 10%, especially 4 to 7%, by weight based on the feedstock. If desired, water may be added, suitably in amounts of up to 20% by weight, although this may, under some circumstances, have the effect of altering the product distribution. In some instances, this altered product distribution may be desirable, and improved selectivity to particular compounds of groups of compounds may be observed.

Preferably, the catalyst is a synthetic alumino-silicate of the type described in our above-mentioned pending patent applications, and more preferably it is a protonic form as specifically described in Application No. 83/30873.

Preferred feedstocks are lower alcohols, ethers and aldehydes, more especially methanol or dimethyl ether. The possibility also exists for the catalyst to be used alone, or in combination with another catalyst, for the direct conversion of synthesis gas or in a process wherein
synthesis gas is converted _in situ_ to methanol or dimethyl ether.

Temperatures used in the method are suitably in the range 300 to
$500^{\circ}$C, expecially 350 to $500^{\circ}$C, and pressures are not critical and may be
at, below or above atmospheric. The total (including additives) feedstock flow rate is suitably a Liquid Hourly Space Velocity (LHSV) of
from 0.1 to $5h^{-1}$, preferably about $1h^{-1}$. The reaction may be carried
out in any suitable reactor, that is fixed bed, fluidised bed or moving
bed reactors.

                              EXAMPLE

20g samples of a 125 micron mean particle size synthetic alumino-
silicate microporous catalyst having a majority of pores of 1.0nm, and
prepared by reacting a hydrolysable aluminium compound with a dry amorphous microporous silica xerogel in an amount to give a monolayer of the
aluminium compound on 50% of the BET surface area of the silica  and
substituting all labile aluminium cations by protons, were packed into
stainless steel reactor tubes of 5mm internal diameter and 250mm length.
In turn, the reactor tubes were connected to a feedstock feed pump set
to give a LHSV of $1h^{-1}$ and to a product collection system for gases and
a cooled liquid collection vessel. The reactor tubes were wrapped with
electrical heating tape and the temperature controlled at $450^{\circ}$C using a
Eurotherm proportional controller. A number of additives were co-fed
with methanol and passed through a reactor tube until the overall conver-
sion of methanol had declined to 50%. The resulting "catalyst life" is
shown in Table 1 below.

0153536

TABLE 1

| Additive | Benzene | | Toluene | | p-xylene | Cyclohexene |
|---|---|---|---|---|---|---|
| Amount of additive | 1% | 5% | 1% | 5% | 5% | 5% |
| Catalyst life (hours) | 6 | 24 | 18 | 28 | 30 | 100 |

The cyclohexene additive gave a substantial improvement in catalyst life. The run was terminated at 100 hours, at which point the methanol conversion had not dropped below 50%.

The products from each methanol additive blend at the 5% level and after 8 hours on stream, were analysed by conventional gas chromatographic and mass spectrometry techniques. The results obtained are given in Table 2. At longer times on stream, although the conversion was reduced, the product distribution was not materially affected.

- 6 -

0153536

TABLE 2

| Composition of Products | | Additive (5%) | | | |
|---|---|---|---|---|---|
| | | Benzene | Toluene | p-Xylene | Cyclohexene |
| Cycloalkanes/ alkanes | (%) | 16.5 | 13.2 | 14.2 | 20.2 |
| Toluene | (%) | 8.9 | 11.8 | 9.1 | 6.9 |
| $C_2$-Alkyl benzenes | (%) | 14.7 | 17.0 | 20.4 | 6.8 |
| $C_3$-Alkyl benzenes | (%) | 18.1 | 15.2 | 19.2 | 18.4 |
| $C_4$-Alkyl benzenes | (%) | 16.9 | 14.7 | 16.9 | 16.8 |
| $C_5$-Alkyl benzenes | (%) | 8.9 | 9.2 | 9.8 | 6.6 |
| $C_6$-Alkyl benzenes | (%) | – | 1.2 | – | 6.1 |
| $C_7$-Alkyl benzenes | (%) | – | – | – | – |
| Dimethyl ether | (%) | 9.1 | 5.8 | 2.1 | 4.9 |
| Methane | (%) | 2.4 | 8.1 | 5.7 | 10.3 |
| Ethane | (%) | 0.4 | 0.5 | 0.3 | 0.5 |
| Higher alkanes & alkenes | (%) | 1.8 | 1.3 | 1.7 | 1.4 |
| Carbon monoxide | (%) | 2.3 | 1.1 | 0.6 | 1.1 |
| % Conversion of methanol | (%) | 93.4 | 92.9 | 91.8 | 100.0 |

Similar results to those obtained with cyclohexene were obtained when cyclohexadiene was substituted therefor in the above test.

Case 4662

PATENT CLAIMS:

1.      A method for converting an oxygen-containing aliphatic feedstock into hydrocarbons, in the vapour phase, over a synthetic amorphous microporous catalyst, characterised by the admixture of from 1 to 15% by weight of an organic additive which contains at least one olefinic linkage which is not deactivated or a precursor thereof, with the oxygen-containing aliphatic feedstock, optionally with the addition of up to 20% by weight of water, based on the feedstock, whereby deactivation of the catalyst is reduced.

2.      A method according to claim 1, characterised in that the amount of additive is from 3 to 10% by weight of the feedstock.

3.      A method according to claim 2, characterised in that the amount of additive is from 4 to 7% by weight of the feedstock.

4.      A method according to claim 1, 2 or 3, characterised in that the feedstock is a lower alcohol, ether or aldehyde.

5.      A method according to claim 4, characterised in that the feedstock is methanol or dimethyl ether.

6.      A method according to any one of the preceding claims, characterised in that the temperature for the conversion reaction is in the range 300 to 550°C.

7.      A method according to any one of the preceding claims, characterised in that the additive is selected from cyclohexene, cyclohexadiene, 1-hexene, butadiene, cyclohexanone and cyclohexanol.

8.      A method according to any one of claims 1 to 7, characterised in that the additive is ethylene or a methyl-substituted derivative thereof.

9.      A method according to any one of the preceding claims, characterised in that the catalyst is a synthetic alumino-silicate catalyst comprising a highly porous amorphous silica xerogel having a layer of an aluminium compound chemically bonded onto the silica surface in an amount equivalent to a monolayer on up to 90% of the BET surface area of the silica, the catalyst having a maximum pore diameter of about 1.1nm.

10. A method according to claim 9, chsracterised in that the catalyst has substantially only protons and/or transition metal cations as electrical charge balancing species on the surface of the layer.

11. A method according to claim 10, characterised in that the aluminium compound is present on the catalyst in an amount equivalent to a monolayer on about 50% of the BET surface area of the silica.